(19) Europäisches Patentamt
European Patent Office
Office européen des brevets

(11) **EP 2 648 640 B1**

(12) **EUROPÄISCHE PATENTSCHRIFT**

(45) Veröffentlichungstag und Bekanntmachung des Hinweises auf die Patenterteilung:
**20.04.2016 Patentblatt 2016/16**

(51) Int Cl.:
*A61B 34/30* (2016.01)          *B25J 9/16* (2006.01)
*G06F 19/00* (2011.01)          *A61B 34/35* (2016.01)

(21) Anmeldenummer: **11791273.3**

(22) Anmeldetag: **01.12.2011**

(86) Internationale Anmeldenummer:
**PCT/EP2011/071476**

(87) Internationale Veröffentlichungsnummer:
**WO 2012/076390 (14.06.2012 Gazette 2012/24)**

(54) **TELEPRÄSENZSYSTEM**

TELEPRESENCE SYSTEM

SYSTÈME DE TÉLÉPRÉSENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.12.2010 DE 102010062648**

(43) Veröffentlichungstag der Anmeldung:
**16.10.2013 Patentblatt 2013/42**

(73) Patentinhaber: **KUKA Roboter GmbH**
**86165 Augsburg (DE)**

(72) Erfinder:
• **JACOB, Dirk**
**87616 Marktoberdorf (DE)**
• **UEBERLE, Marc-Walter**
**86316 Friedberg (DE)**
• **NEFF, Thomas**
**80339 München (DE)**
• **KUSCHEL, Martin**
**80339 München (DE)**

• **ORTMAIER, Tobias**
**30966 Hemmingen (DE)**

(74) Vertreter: **Patentanwälte**
**Funk & Böss GbR**
**Sigmundstraße 1**
**80538 München (DE)**

(56) Entgegenhaltungen:
**DE-A1-102004 012 042     US-A- 5 046 022**
**US-A1- 2007 010 256**

• **DONGJUN LEE ET AL: "Bilateral Teleoperation of Multiple Cooperative Robots over Delayed Communication Networks: Application", ROBOTICS AND AUTOMATION, 2005. PROCEEDINGS OF THE 2005 IEEE INTERNATIONAL CONFERENCE ON BARCELONA, SPAIN 18-22 APRIL 2005, PISCATAWAY, NJ, USA,IEEE, 18. April 2005 (2005-04-18), Seiten 366-371, XP010872167, DOI: 10.1109/ROBOT.2005.1570146 ISBN: 978-0-7803-8914-4**

**EP 2 648 640 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Telepräsenzsystem.

In der Telepräsenz bzw. Teleoperation werden Roboter über relativ große Distanzen ferngesteuert. Dabei werden Sollkommandos von einer Bedienperson an einer Bedienvorrichtung bzw. Eingabekonsole sensoriell erfasst, verarbeitet und an einen entfernt stehenden Teleoperator übertragen. Eine Anwendung solcher Systeme ist die telemanipulierte Chirurgie, bei der z.B. ein Arzt mittels seiner Eingabekonsole manuell mehrere Roboterarme bewegt, die z.B. ein medizinisches Instrument bewegen, um ein Lebewesen damit zu behandeln, insbesondere zu operieren. Die WO 2010/025943 A1 und US-A-5046022 offenbaren ein Beispiel eines solchen medizinischen Arbeitsplatzes.

Weiterhin ist aus der DE-A-102004012042 bekannt, einen Puffer zur Übertragung von Daten zu verwenden, um die Stabilität der Datenübertragung zu verbessern.

Die Aufgabe der Erfindung ist es, ein verbessertes Telepräsenzsystem anzugeben.

Die Aufgabe der Erfindung wird gelöst durch ein Telepräsenzsystem, aufweisend

- eine Mensch-Maschine Schnittstelle,
- einen Teleoperator, der eingerichtet ist, über einen Kommunikationskanal mit der Mensch-Maschine Schnittstelle bidirektional zu kommunizieren, und der eingerichtet ist, aufgrund von über den Kommunikationskanal übertragener und aufgrund einer manuellen Betätigung der Mensch-Maschine Schnittstelle erzeugter erster Signale eine Aktion auszuführen, aufgrund der ausgeführten Aktion auf seine Umgebung zu reagieren und, als Antwort auf das Reagieren auf seine Umgebung, zugeordnete zweite Signale über den Kommunikationskanal an die Mensch-Maschine Schnittstelle zu senden, und
- wenigstens eine Puffervorrichtung, die eingerichtet ist, die über den Kommunikationskanal übertragenen Signale derart zu puffern und zeitverzögert abzugeben, sodass die von der Mensch-Maschine Schnittstelle und die von dem Teleoperator stammenden Signale jeweils mit einer effektiven konstanten Zeitdauer zeitverzögert über den Kommunikationskanal übertragen werden.

Das erfindungsgemäße Telepräsenzsystem ist derart ausgeführt, dass eine Person mittels der Mensch-Maschine Schnittstelle über den Kommunikationskanal den Teleoperator bedienen kann. Der Teleoperator ist z. B. als wenigstens ein Roboterarm mit mehreren, mittels Gelenken verbundenen Gliedern, Antrieben zum Bewegen der Glieder, und einer Befestigungsvorrichtung, sowie einer Steuervorrichtung zum Ansteuern der Antriebe des wenigstens einen Roboterarms ausgebildet.

Im Betrieb des erfindungsgemäßen Telepräsenzsystems reagiert der Teleoperator mit seiner Umgebung z.B. in der Form, dass wenn er einen Gegenstand anfährt, eine Kraft auf den Teleoperator wirkt. Diese Information wird der Mensch-Maschine Schnittstelle über den Kommunikationskanal übermittelt, sodass diese der Person z.B. eine haptische Rückmeldung geben kann.

Insbesondere aus Stabilitätsgründen kann es erstrebenswert ein, dass der Kommunikationskanal Signale in Richtung Teleoperator mit derselben Zeitdauer verzögert wie Signale in Richtung Mensch-Maschine Schnittstelle. Um dies sicher zu stellen, umfasst das erfindungsgemäße Telepräsenzsystem die wenigstens eine Puffervorrichtung, die eingerichtet ist, die über den Kommunikationskanal übertragenen Signale derart zu puffern und zeitverzögert abzugeben, sodass die von der Mensch-Maschine Schnittstelle und die von dem Teleoperator stammenden Signale jeweils mit einer effektiven konstanten Zeitdauer zeitverzögert über den Kommunikationskanal übertragen werden. Vorzugsweise, wie dies nach einer Variante des erfindungsgemäßen Telepräsenzsystems vorgesehen ist, ist die wenigstens eine Puffervorrichtung derart eingerichtet, die über den Kommunikationskanal übertragenen Signale derart zu puffern und zeitverzögert abzugeben, sodass die von der Mensch-Maschine Schnittstelle und die von dem Teleoperator stammenden Signale effektiv mit derselben Zeitdauer zeitverzögert über den Kommunikationskanal übertragen werden.

Dadurch wird es auch bei relativ großen unterschiedlichen Zeitverzögerungen des bidirektionalen Kommunikationskanals möglich, wie dies nach einer bevorzugten Ausführungsform des erfindungsgemäßen Telepräsenzsystems vorgesehen ist, dass dessen Mensch-Maschine Schnittstelle derart ausgeführt ist, dass sie die aufgrund der manuellen Betätigung der Mensch-Maschine Schnittstelle erzeugten ersten Signale einer passivierenden Transformation, insbesondere einer Scattering-Transformation unterzieht, um erste transformierte Signale zu erhalten und diese über den Kommunikationskanal an den Teleoperator zu übermitteln, der Teleoperator eingerichtet ist, die empfangenen ersten transformierten Signale rück zu transformieren, um die Aktion auszuführen, die zweiten Signale einer passivierenden Transformation, insbesondere einer Scattering-Transformation zu unterziehen, um zweite transformierte Signale zu erhalten und diese über den Kommunikationskanal an die Mensch-Maschine Schnittstelle zu übermitteln, und die Mensch-Maschine Schnittstelle eingerichtet ist, die empfangenen zweiten transformierten Signale rück zu transformieren. Die Scattering-Transforamtion ist dem Fachmann als solche bekannt und dient dazu, einen Kommunikationskanal zu passivieren, wodurch die Stabilitätsreserven des erfindungsgemäßen Telepräsenzsystems erhöht werden.

Die wenigstens eine Puffervorrichtung kann z. B. derart ausgeführt sein, dass sie die über den Kommunikationskanal übermittelten Signale puffert und zeitverzögert abgibt. Die Puffervorrichtung kann z.B. in den Teleoperator oder in die Mensch-Maschine Schnittstelle integriert sein.

Nach einer Variante des erfindungsgemäßen Telepräsenzsystems ist die wenigstens eine Puffervorrichtung derart eingerichtet, dass sie die über den Kommunikationskanal übertragenen Signale derart puffert und zeitverzögert abgibt, sodass die von der Mensch-Maschine Schnittstelle und die von dem Teleoperator stammenden Signale mit derselben Zeitdauer, die einer maximalen, durch den Kommunikationskanal bedingten Zeitverzögerung entspricht, zeitverzögert über den Kommunikationskanal übertragen werden.

Gemäß einer bevorzugten Ausführungsform des erfindungsgemäßen Telepräsenzsystems ist die Puffervorrichtung derart ausgeführt, dass sie die über den Kommunikationskanal übertragenen ersten transformierten Signale puffert und zeitverzögert abgibt, sodass der Teleoperator die von der Puffervorrichtung zeitverzögerten ersten transformierten Signale rück transformiert. Gemäß dieser Variante umfasst das erfindungsgemäße Telepräsenzsystem eine weitere Puffervorrichtung, die die über den Kommunikationskanal übertragenen zweiten transformierten Signale puffert und zeitverzögert abgibt, sodass die Mensch-Maschine Schnittstelle die von der weiteren Puffervorrichtung zeitverzögerten zweiten transformierten Signale zurück transformiert. Somit ist es möglich, je nachdem in welche Richtung der Kommunikationskanal übertragene Signale stärker verzögert stets Signale derart zu puffern und zeitverzögert abzugeben, dass die von der Mensch-Maschine Schnittstelle und die von dem Teleoperator stammenden Signale mit derselben Zeitdauer zeitverzögert über den Kommunikationskanal übertragen werden. Die Zeitverzögerung *PT1* der Puffervorrichtung und die Zeitverzögerung *PT2* der weiteren Puffervorrichtung können folgendermaßen ermittelt werden:

$$PT2 = Tmax - T1$$

$$PT1 = Tmax - T2.$$

wobei *Tmax* wenigstens die maximale Zeitverzögerung des Kommunikationskanals ist, und *T1* die Zeitverzögerung des Kommunikationskanals in Richtung Mensch-Maschine Schnittstelle / Teleoperator und T2 die Zeitverzögerung des Kommunikationskanals in Richtung Teleoperator / Mensch-Maschine Schnittstelle ist.

Das erfindungsgemäße Telepräsenzsystem kann derart eingerichtet sein, die Zeitverzögerungen des Kommunikationskanals mittels Zeitmessung an der Mensch-Maschine Schnittstelle und dem Teleoperator automatisch zu ermitteln.

Für eine Synchronisation des erfindungsgemäßen Telepräsenzsystems kann es vorgesehen sein, dass dieses eingerichtet ist, den Takt des Kommunikationskanals für von der Mensch-Maschine Schnittstelle gesendeter Signale mittels des Takts der Mensch-Maschine Schnittstelle zu triggern, den Takt des Teleoperators mittels des Takts des Kommunikationskanals für in Richtung Teleoperator gesendeter Signale zu triggern, und den Takt des Kommunikationskanals in Richtung Mensch-Maschine Schnittstelle mit dem Takt des Teleoperators zu triggern. Das erfindungsgemäße Telepräsenzsystem kann ferner derart eingerichtet sein, den Takt des Kommunikationskanals in Richtung Mensch-Maschine Schnittstelle gesendeter Signale und den Takt der Mensch-Maschine Schnittstelle zu vergleichen und aufgrund des Vergleichs auf ein Fehlen einer Synchronisation des Telepräsenzsystems zu schließen. Sind z. B. diese beiden Takte nicht synchron bzw. nicht in Phase, dann kann auf ein Fehlen einer Synchronisation geschlossen werden.

Das erfindungsgemäße Telepräsenzsystem ist insbesondere als ein medizinischer Arbeitsplatz ausgebildet.

Je nach Ausgestaltung des erfindungsgemäßen Telepräsenzsystems, das auch als Teleoperationssystem bezeichnet werden kann, wird ein menschlicher Bediener (Operator) befähigt, in einer entfernten Umgebung mittels des Teloperators aktiv zu werden. Dabei kommandiert der Bediener über die Mensch-Maschine Schnittstelle den Teleoperator, der beispielsweise als ein Roboter ausgeführt ist, in der entfernten Umgebung.

Das erfindungsgemäße Telepräsenzsystem ist bidirektional ausgeführt, d.h. es werden nicht nur Signale von der Mensch-Maschine Schnittstelle zum Teleoperator gesendet, sondern auch in die entgegen gesetzte Richtung. Der Teleoperator misst z.B. verschiedene Umgebungswerte und reflektiert diese zur Mensch-Maschine Schnittstelle, die insbesondere die entfernte Umgebung dem Operator darstellt. Kommandierte- und reflektierte Signale werden dabei über den Kommunikationskanal, z.B. dem Internet, ausgetauscht.

Das erfindungsgemäße Telepräsenzsystem ist insbesondere als ein bilaterales, kinästhetisch-haptisches Telepräsenzsystem ausgebildet, bei dem insbesondere Kraft- und Geschwindigkeitssignale ausgetauscht werden. Dabei wird beispielsweise ein energiebehafteter Regelkreis zwischen Operator und Teleoperator geschlossen. Verzögerungen im Kommunikationskanal können zur Instabilität des gesamten Systems führen und werden vorzugsweise durch eine passivierende Transformation, insbesondere mittels der Scattering-Transformation, stabilisiert.

Ein Ausführungsbeispiel der Erfindung ist exemplarisch in den beigefügten schematischen Figuren dargestellt. Es zeigen:

Fig. 1     ein Prinzipschaltbild eines Telepräsenzsystems,

Fig. 2     einen medizinischen Arbeitsplatz als Beispiels eines Telepräsenzsystems und

Fig. 3    ein Blockschaltbild zur Veranschaulichung einer für das Telepräsenzsystem durchgeführten Synchronisation und Verifikation.

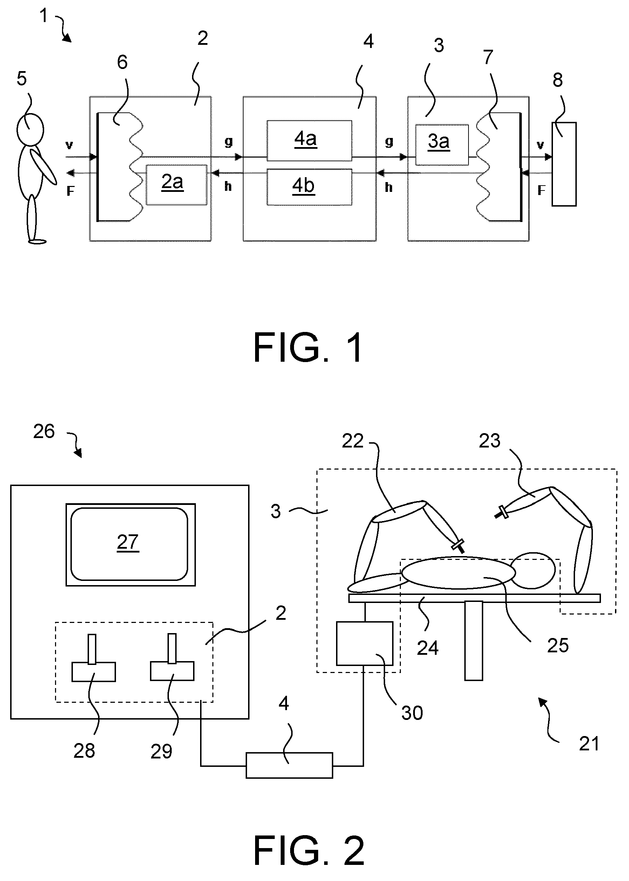

Die Fig. 1 zeigt ein Prinzipschaltbild eines Telepräsenzsystems 1, das z.B. als ein in der Fig. 2 gezeigter medizinischer Arbeitsplatz 21 ausgeführt sein kann.

Das Telepräsenzsystem 1 weist eine Mensch-Maschinen Schnittstelle 2 und einen Teleoperator 3 auf, die über einen Kommunikationskanal 4 miteinander kommunizieren. Der Kommunikationskanal 4 ist beispielsweise ein Datenübertragungsnetz, z.B. das Internet.

Im Falle des vorliegenden Ausführungsbeispiels ist der Teleoperator 3 als eine Steuervorrichtung 30 und mehrere Roboterarme 22, 23 ausgeführt, die jeweils mehrere, über Gelenke verbundene Glieder aufweisen und deren Bewegungen mittels der mit dem Kommunikationskanal 4 verbundenen Steuervorrichtung 30 gesteuert oder auch geregelt werden. Mittels der Roboterarme 22, 23 kann z.B. ein auf einer Patientenliege 24 liegendes Lebewesen 25 behandelt werden.

Im Falle des vorliegenden Ausführungsbeispiels umfasst der medizinische Arbeitsplatz 21 eine Bedienkonsole 26, die beispielsweise eine Anzeigevorrichtung 27 und manuelle Eingabevorrichtungen 28, 29 aufweist, mittels derer eine Person 5, z.B. ein Chirurg, die Roboterarme 22, 23 telemanipulieren kann. Die beiden Eingabevorrichtung 28, 29 sind ein Beispiels der Mensch-Maschine Schnittstelle 2 des Telepräsenzsystems 1.

Bei einer manuellen Bewegung der Eingabevorrichtungen 28, 29 durch die Person 5 werden z.B. die Eingabevorrichtungen 28, 29 mit einer Geschwindigkeit $v$ bewegt, wodurch die Eingabevorrichtungen 28, 29 bzw. die Mensch-Maschinen Schnittstelle 2 entsprechende Signale erzeugt, welche eine Information über die Geschwindigkeit $v$ aufweisen.

Im Falle des vorliegenden Ausführungsbeispiels ist die Mensch-Maschinen Schnittstelle 2 derart ausgeführt, dass diese die Geschwindigkeit $v$ zunächst der sogenannten, dem Fachmann jedoch bekannten Scattering-Transformation unterzieht. Dies ist mittels eines Funktionsblocks 6 der Mensch-Maschine Schnittstelle 2 in de Fig. 1 veranschaulicht. Die Parameter der Scattering-Transformation sind, wie es dem Fachmann im Prinzip bekannt ist, die Parameter $g = \dfrac{b \cdot v + F}{\sqrt{2 \cdot b}}$

und $h = \dfrac{b \cdot v - F}{\sqrt{2 \cdot b}}$ mit frei wählbarer Konstante b.

Die mittels der Scattering-Transformation transformierte Geschwindigkeit wird anschließend über den Kommunikationskanal 4 an die Steuervorrichtung 30, allgemein an den Teleoperator 3, übertragen. In Richtung Mensch-Maschine Schnittstelle 2 / Teleoperator 3 stellt der Kommunikationskanal 4 einen ersten Teilkommunikationskanal 4a mit einer Zeitverzögerung (Kommunikationslatenz) $T1$ dar.

Im Falle des vorliegenden Ausführungsbeispiels ist die Steuervorrichtung 30 bzw. der Teleoperator 3 derart ausgeführt, dass er zunächst die über den Teilkommunikationskanal 4a erhaltene transformierte Geschwindigkeit z.B. mittels eines Puffers 3a zwischenspeichert und zeitverzögert die transformierte Geschwindigkeit zurück transformiert, sodass sich z.B. der relevante Roboterarm 22, 23, gesteuert durch die Steuervorrichtung 30, entsprechend der manuellen Bewegung der Eingabevorrichtungen 28, 29 bewegt. Die Rücktransformation ist mittels eines Funktionsblocks 7 des Teleoperators 3 in der Fig. 1 veranschaulicht. Der Puffer 3a des Teleoperators 3 verzögert im Falle des vorliegenden Ausführungsbeispiels das der transformierten Geschwindigkeit zugeordnete Signal um eine Zeitdauer $PT1$.

Im Falle des vorliegenden Ausführungsbeispiels interagiert der Teleoperator 3 mit der im Vergleich zur Mensch-Maschine Schnittstelle 2 entfernten Umgebung 8 z.B. derart, dass der relevante Roboterarm 22, 23 ein medizinisches Instrument in das Lebewesen 25 einführt. Aufgrund dieser Interaktion erfährt der Teleoperator 3 z.B. eine Kraft F, deren zugeordnetes Signal der Teleoperator 3 mittels des Funktionsblocks 7 einer Scattering-Transforamtion unterzieht.

Die mittels der Scattering-Transformation transformierte Kraft wird anschließend über den Kommunikationskanal 4 an die Eingabevorrichtungen 28, 29, allgemein an die Mensch-Maschine Schnittstelle 2 übertragen. In Richtung Teleoperator 3 / Mensch-Maschine Schnittstelle 2 stellt der Kommunikationskanal 4 einen zweiten Teilkommunikationskanal 4b mit einer Zeitverzögerung (Kommunikationslatenz bzw. Kommunikationsverzögerung) T2 dar.

Im Falle des vorliegenden Ausführungsbeispiels ist die Mensch-Maschine Schnittstelle 2 derart ausgeführt, dass sie zunächst die über den Teilkommunikationskanal 4b erhaltene transformierte Kraft z.B. mittels eines Puffers 2a zwischenspeichert und zeitverzögert die transformierte Kraft zurück transformiert, sodass z.B. die Eingabevorrichtungen 28, 29 eine der Kraft am relevanten Roboterarm 22, 23 haptische Rückmeldung an die Person 5 erzeugen. Die Rücktransformation ist mittels des Funktionsblocks 6 der Mensch-Maschine Schnittstelle 2 in der Fig. 1 veranschaulicht. Der Puffer 2a der Mensch-Maschine Schnittstelle 2 verzögert im Falle des vorliegenden Ausführungsbeispiels das der transformierten Kraft zugeordnete Signal um eine Zeitdauer $PT2$.

Um eine stabile Übertragung der Signale über den Kommunikationskanal 4 zu erhalten, wird dieser mittels der Scattering-Transformation passiviert. Damit dies zufriedenstellend realisiert werden kann, sollte der Kommunikationskanal 4 in beide Richtungen jeweils konstante Zeitverzögerung der übertragenen Signale aufweisen. Vorzugsweise können beide Zeitverzögerungen dieselben sein, d.h. die Zeitverzögerungen der Teilkommunikationskanäle 4a, 4b können gleich und konstant sein. Dies ist bei realen Kommunikationskanälen in der Regel nicht der Fall, da diese in der Realität in der Regel variieren.

Da für das vorliegende Ausführungsbeispiels sowohl die Signale von der Mensch-Maschine-Schnittstelle 2 zum Teleopearator 3 und die Signal vom Teleoperator 3 zur Mensch-Maschine-Schnittstelle 2 für die Übertragung über den Kommunikationskanal 4 der Scattering-Transformation unterzogen werden, ergeben sich im Fall des vorliegenden Ausführungsbeispiels folgende Parameter mit der frei wählbaren Konstante b:

$$g_l = \frac{b \cdot v_{mmi} + F_{mmi}}{\sqrt{2 \cdot b}}$$

$$h_l = \frac{b \cdot v_{mmi} - F_{mmi}}{\sqrt{2 \cdot b}}$$

$$g_r = \frac{b \cdot v_{to} + F_{to}}{\sqrt{2 \cdot b}}$$

$$h_r = \frac{b \cdot v_{to} - F_{to}}{\sqrt{2 \cdot b}}$$

wobei:

$g_1$ die abgehende Scattering Variable (Englisch: "incident wave") auf der Seite der Mensch-Maschine-Schnittstelle 2,

$g_r$ die eintreffende Scattering Variable (Englisch: "reflected wave") auf der Seite des Teleoperators 3,

$h_r$ die abgehende Scattering Variable auf der Seite des Teleoperators 3,

$g_r$ die eintreffende Scattering Variable auf der Seite der Mensch Maschine Schnittstelle 2 ist,

$V_{mmi}$ die Geschwindigkeit der Mensch-Maschine-Schnittstelle 2 und

$v_{to}$ die Geschwindigkeit des Teleoperators 3 ist, und

$F_{mmi}$ die Kraft ist, die durch die Mensch-Maschine Schnittstelle 2 der Person 5 eingeprägt wird, und

$F_{to}$ die Kraft ist, die vom Teleoperator 3 auf die entfernte Umgebung 8 ausgeübt wird.

Zumindest in vielen, wenn nicht gar in den meisten oder allen Fällen kann jedoch eine maximale Zeitverzögerung *Tmax* des Kommunikationskanals 4 bestimmt werden, die insbesondere mit hoher Wahrscheinlichkeit nicht überschritten wird.

Im Falle des vorliegenden Ausführungsbeispiels sind dafür die Pufferungen der erhaltenen Signale an der Mensch-Maschine Schnittstelle 2 bzw. am Teleoperator 3 beispielsweise mittels der Puffer 2a, 3a vorgesehen, die derart das kommandierte sowie das reflektierte Signal verzögern, dass eine konstante Verzögerung erreicht wird. Durch eine Zeitmessung an der Mensch-Maschine Schnittstelle 2 und am Teleoperator 3 und Stempelung von über den Kommunikationskanal 4 übermittelter Kommunikationspakete werden z.B. vor einem Betrieb des medizinischen Arbeitsplatzes 21, allgemein des Telepräsenzsystems 1 die Zeitdauern *T1, T2,* also die Kommunikationslatenzen des Kommunikationskanals 4 bestimmen. Für die Laufzeit kommandierter Pakete, also die Zeitdauer *T1,* die das Datenpaket von der Mensch-Maschine Schnittstelle 2 zum Teleoperator 3 benötigt, wenn es über den Kommunikationskanal 4 übermittelt wird, gilt:

$$T1 = t_2 - t_1$$

wobei $t_2$ der Zeitpunkt ist, an dem das Datenpaket den Teleoperator 3 erreicht, und $t_1$ der Zeitpunkt ist, an dem das Datenpaket die Mensch-Maschine Schnittstelle 2 verlässt.

Für die Laufzeit reflektierter Datenpakete, also die Zeitdauer *T2,* die das Datenpaket vom Teleoperator 3 zur Mensch-Maschine Schnittstelle 2 benötigt, wenn es über den Kommunikationskanal 4 übermittelt wird, gilt:

$$T2 = t_4 - t_3$$

wobei $t_3$ der Zeitpunkt ist, an dem das Datenpaket den Teleoperator 3 verlässt, und $t_4$ der Zeitpunkt ist, an dem das Datenpaket die Mensch-Maschine Schnittstelle 2 erreicht.

Mit Angabe der maximale Zeitverzögerung *Tmax* des Kommunikationskanals 4 und den Zeitdauern *T1*, *T2* lassen sich die Längen der Pufferungen (Zeitdauern *PT1, PT2*) der Puffer 2a, 3a bestimmen:

$$PT2 = Tmax - T1$$

$$PT1 = Tmax - T2.$$

Als Resultat der Synchronisation und der Pufferungen entsteht eine konstante resultierende Kommunikationslatenz des Kommunikationskanals 4 in beide Richtungen, die der maximale Zeitverzögerung *Tmax* entspricht.

Die maximale Zeitverzögerung *Tmax* wird dann als ein Parameter der beiden Scattering-Transformationen verwendet.

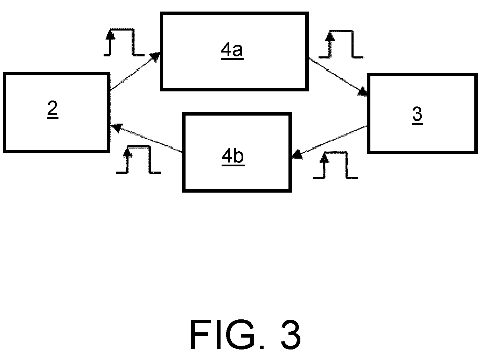

Im Falle des vorliegenden Ausführungsbeispiels werden die Mensch-Maschine Schnittstelle 2, der Teleoperator 3 und der Kommunikationskanal 4 synchronisiert. Die Fig. 3 veranschaulich die Synchronisation der Bussysteme von Mensch-Maschine Schnittstelle 2, Kommunikationskanal 4 und Teleoperator 3.

Zunächst wird der Takt der Mensch-Maschine Schnittstelle 2 als Referenztakt heran gezogen. Über diesen wird zunächst der Takt des Teilkommunikationskanals 4a des Kommunikationskanals 4 für das kommandierte Signal, also für das für den Teleoperator 3 bestimmte Signal getriggert. Dieser wirkt daraufhin als Taktgeber für den Takt des Teleoperators 3. Der Takt des Teleoperators 3 wiederum triggert den Takt des Teilkommunikationskanals 4b des Kommunikationskanals 4 für das reflektierte Signal, also für das vom Teleoperator 3 erzeugte und für die Mensch-Maschine Schnittstelle 2 bestimmte Signal.

Der Takt des Teilkommunikationskanals 4b des Kommunikationskanals 4 wird daraufhin mit dem Referenztakt, also mit dem Takt der Mensch-Maschine Schnittstelle 2 verglichen. Bei einem Phasenunterschied wird auf einen Fehler bei der Synchronisation der verschiedenen Takte geschlossen.

## Patentansprüche

1. Telepräsenzsystem, aufweisend

   - eine Mensch-Maschine Schnittstelle (2), und
   - einen Teleoperator (3), der eingerichtet ist, über einen Kommunikationskanal (4) mit der Mensch-Maschine Schnittstelle (2) bidirektional zu kommunizieren, und der eingerichtet ist, aufgrund von über den Kommunikationskanal (4) übertragener und aufgrund einer manuellen Betätigung der Mensch-Maschine Schnittstelle (2) erzeugter erster Signale eine Aktion auszuführen, aufgrund der ausgeführten Aktion auf seine Umgebung zu reagieren und, als Antwort auf das Reagieren auf seine Umgebung, zugeordnete zweite Signale über den Kommunikationskanal (4) an die Mensch-Maschine Schnittstelle (2) zu senden, **gekennzeichnet durch**
   - wenigstens eine Puffervorrichtung (2a, 3a), die eingerichtet ist, die über den Kommunikationskanal (4) übertragenen Signale derart zu puffern und zeitverzögert abzugeben, sodass die von der Mensch-Maschine Schnittstelle (2) und die von dem Teleoperator (3) stammenden Signale jeweils mit einer effektiven konstanten Zeitdauer zeitverzögert über den Kommunikationskanal (4) übertragen werden.

2. Telepräsenzsystem, dessen wenigstens eine Puffervorrichtung (2a, 3a) eingerichtet ist, die über den Kommunikationskanal (4) übertragenen Signale derart zu puffern und zeitverzögert abzugeben, sodass die von der Mensch-Maschine Schnittstelle (2) und die von dem Teleoperator (3) stammenden Signale effektiv mit derselben Zeitdauer zeitverzögert über den Kommunikationskanal (4) übertragen werden.

3. Telepräsenzsystem nach Anspruch 1 oder 2, dessen Mensch-Maschine Schnittstelle (2) derart ausgeführt ist, dass sie die aufgrund der manuellen Betätigung der Mensch-Maschine Schnittstelle (2) erzeugten ersten Signale einer passivierenden Transformation, insbesondere einer Scattering-Transformation unterzieht, um erste transformierte Signale zu erhalten und diese über den Kommunikationskanal (4) an den Teleoperator (3) zu übermitteln, der Teleoperator (3) eingerichtet ist, die empfangenen ersten transformierten Signale rück zu transformieren, um die Aktion auszuführen, die zweiten Signale einer passivierenden Transformation, insbesondere einer Scattering-Transformation zu unterziehen, um zweite transformierte Signale zu erhalten und diese über den Kommunikationskanal (4) an die Mensch-Maschine Schnittstelle (2) zu übermitteln, und die Mensch-Maschine Schnittstelle (2) eingerichtet ist, die empfangenen zweiten transformierten Signale rück zu transformieren.

4. Telepräsenzsystem nach einem der Ansprüche 1 bis 3, bei dem die wenigstens eine Puffervorrichtung (2a, 3a) die über den Kommunikationskanal (4) übermittelten Signale puffert und zeitverzögert abgibt.

5. Telepräsenzsystem nach einem der Ansprüche 1 bis 4, bei dem die wenigstens eine Puffervorrichtung (2a, 3a) derart eingerichtet ist, die über den Kommunikationskanal (4) übertragenen Signale derart zu puffern und zeitverzögert abzugeben, sodass die von der Mensch-Maschine Schnittstelle (2) und die von dem Teleoperator (3) stammenden Signale mit derselben Zeitdauer, die einer maximalen, durch den Kommunikationskanal (4) bedingten Zeitverzögerung entspricht, zeitverzögert über den Kommunikationskanal (4) übertragen werden.

6. Telepräsenzsystem nach Anspruch 3, bei dem die Puffervorrichtung (3a) die über den Kommunikationskanal (4) übertragenen ersten transformierten Signale puffert und zeitverzögert abgibt, sodass der Teleoperator (3) die von der Puffervorrichtung (3a) zeitverzögerten ersten transformierten Signale rück transformiert, und eine weitere Puffervorrichtung (2a) vorgesehen ist, die die über den Kommunikationskanal (4) übertragenen zweiten transformierten Signale puffert und zeitverzögert abgibt, sodass die Mensch-Maschine Schnittstelle (2) die von der weiteren Puffervorrichtung (2a) zeitverzögerten zweiten transformierten Signale zurück transformiert.

7. Telepräsenzsystem nach Anspruch 6, bei dem die Zeitverzögerung *PT1* der Puffervorrichtung (3a) und die Zeitverzögerung *PT2* der weiteren Puffervorrichtung (2a) folgendermaßen berechnen:

$$PT2 = Tmax - T1$$

$$PT1 = Tmax - T2.$$

wobei *Tmax* wenigstens die maximale Zeitverzögerung des Kommunikationskanals (4) ist, und *T1* die Zeitverzögerung des Kommunikationskanals (4) in Richtung Mensch-Maschine Schnittstelle (2) / Teleoperator (3) und T2 die Zeitverzögerung des Kommunikationskanals (4) in Richtung Teleoperator (3) / Mensch-Maschine Schnittstelle (2) ist.

8. Telepräsenzsystem nach Anspruch 7, das eingerichtet ist, die Zeitverzögerungen des Kommunikationskanals (4) mittels Zeitmessung an der Mensch-Maschine Schnittstelle (2) und dem Teleoperator (3) zu ermitteln.

9. Telepräsenzsystem nach einem der Ansprüche 1 bis 8, das eingerichtet ist, den Takt des Kommunikationskanals (4) für von der Mensch-Maschine Schnittstelle (2) gesendeter Signale mittels des Takts der Mensch-Maschine Schnittstelle (2) zu triggern, den Takt des Teleoperators (3) mittels des Takts des Kommunikationskanals (4) für in Richtung Teleoperator (3) gesendeter Signale zu triggern, den Takt des Kommunikationskanals (4) in Richtung Mensch-Maschine Schnittstelle (2) mit dem Takt des Teleoperators (3) zu triggern, und aufgrund eines Vergleichs des Takts des Kommunikationskanals (4) in Richtung Mensch-Maschine Schnittstelle (2) gesendeter Signale und des Takts der Mensch-Maschine Schnittstelle (2) auf ein Fehlen einer Synchronisation des Telepräsenzsystems (1) zu schließen

10. Telepräsenzsystem nach einem der Ansprüche 1 bis 9, bei dem der Teleoperator (3) als wenigstens ein Roboterarm (22, 23) mit mehreren, mittels Gelenken verbundenen Gliedern, Antrieben zum Bewegen der Glieder, und einer Befestigungsvorrichtung, sowie einer Steuervorrichtung (30) zum Ansteuern der Antriebe des wenigstens einen Roboterarms (22, 23) ausgebildet ist.

11. Telepräsenzsystem nach einem der Ansprüche 1 bis 10, das als ein medizinischer Arbeitsplatz ausgebildet ist.

**Claims**

1. Telepresence system comprising:

   - a man-machine interface (2), and
   - a teleoperator (3), which is designed to bi-directionally communicate via a communication channel (4) with the man-machine interface (2), and which is designed to perform an action on the basis of first signals transmitted via the communication channel (4) and generated by a manual operation of the man-machine interface (2), to respond to its environment based on the action performed and to send second signals via the communication channel (4) to the man-machine interface (2) in response to the reaction to its environment, **characterised by**
   - at least one buffer device (2a, 3a), which is set up to buffer the signals transmitted via the communication channel (4) and to emit them time-delayed, so that the signals coming from the man-machine interface (2) and the signals coming from the teleoperator (3) are each transmitted time-delayed via the communication channel (4) with an effective constant time period.

2. Telepresence system, whose at least one buffer device (2a, 3a) is set up to buffer the signals transmitted via the communication channel (4) and to emit them time delayed, so that the signals coming from the man-machine interface (2) and the signals coming from the teleoperator (3) are effectively transmitted time-delayed via the communication channel (4) with the same time period.

3. Telepresence system according to either claim 1 or claim 2, whose man-machine interface (2) is designed in such a manner that it subjects the first signals, which are generated as a result of the manual operation of the man-machine interface, to a passivating transformation, in particular to a scattering transformation, in order to obtain first transformed signals and to send the latter via the communication channel (4) to the teleoperator (3), wherein the teleoperator (3) is set up to inversely transform the received first transformed signals in order to execute the action, to subject second signals to a passivating transformation, in particular a scattering transformation, to obtain second transformed signals and to send the latter via the communication channel (4) to the man-machine interface (2), and the man-machine interface (2) is designed to inversely transform the received second transformed signals.

4. Telepresence system according to any one of claims 1 to 3, wherein the at least one buffer device (2a, 3a) buffers the signals transmitted via the communication channel (4) and emits them time delayed.

5. Telepresence system according to any one of claims 1 to 4, wherein the at least one buffer device (2a, 3a) is set up to buffer the signals transmitted via the communication channel (4) and emit them time delayed, so that the signals coming from the man-machine interface (2) and the signals coming from the teleoperator (3) are transmitted time-delayed via the communication channel (4) with the same time period, which corresponds to a maximum time delay dependent on the communication channel (4).

6. Telepresence system according to claim 3, wherein the buffer device (3a) buffers the first transformed signals transmitted via the communication channel (4) and emits them time delayed, so that the teleoperator (3) inversely transforms the first transformed signals delayed by the buffer device (3a), and another buffer device (2a) is provided for buffering the second transformed signals transmitted via the communication channel and emits them time delayed, so that the man-machine interface (2) inversely transforms the second transformed signals delayed by the additional buffer device (2a).

7. Telepresence system according to claim 6, wherein the time delay *PT1* of the buffer device (3a) and the time delay *PT2* of the further buffer device (2a) are calculated as follows:

$$PT2 = Tmax - T1$$

$$PT1 = Tmax - T2,$$

where *Tmax* is at least the maximum time delay of the communication channel (4), and *T1* is the time delay of the communication channel (4) in the direction of man-machine interface (2) / teleoperator (3), and *T2* is the time delay of the communication channel (4) in the direction of teleoperator (3) / human-machine interface (2).

8. Telepresence system according to claim 7, which is set up to determine the time delays of the communication channel (4) by measuring the time at the man-machine interface (2) and the teleoperator (3).

9. Telepresence system according to any one of claims 1 to 8, which is set up to trigger the clock of the communication channel (4) for signals sent by the man-machine interface (2) by means of the clock of the man-machine interface (2), to trigger the clock of the teleoperator (3) by means of the clock of the communication channel (4) for signals transmitted in the direction of the teleoperator (3), to trigger the clock of the communication channel (4) in the direction of the man-machine interface (2) by the clock of the teleoperator (3), and by comparing the clock of the communication channel (4) of signals transmitted in the direction of man-machine interface and the clock of the man-machine interface (2) to detect a lack of synchronization of the telepresence system (1).

10. Telepresence system according to any one of claims 1 to 9, wherein the teleoperator (3) is designed as at least one robot arm (22, 23) with a plurality of links connected by joints, drives for moving the links, and a fastening device, and a controller (30) for controlling the drives of the at least one robot arm (22, 23).

11. Telepresence system according to any one of claims 1 to 10, which is designed as a medical workstation.

**Revendications**

1. Système de téléprésence, comportant :

    - une interface homme-machine (2) et
    - un téléopérateur (3) qui est prévu pour communiquer de façon bidirectionnelle avec l'interface homme-machine (2) via un canal de communication (4), et qui est prévu pour exécuter une action sur la base de premiers signaux transmis via le canal de communication (4) et engendrés sur la base d'un actionnement manuel de l'interface homme-machine (2), pour réagir à son environnement sur la base de l'action exécutée et, en réponse à la réaction à son environnement, pour envoyer des deuxièmes signaux associés à l'interface homme-machine (2) via le canal de communication (4) **caractérisé par**
    - au moins un dispositif tampon (2a, 3a) qui est prévu pour bufferiser et émettre avec retard de temps les signaux transmis via le canal de communication (4) de telle sorte que les signaux provenant de l'interface homme-machine (2) et du téléopérateur (3) sont chacun transmis avec retard de temps avec une durée constante effective via le canal de communication (4).

2. Système de téléprésence dont au moins un dispositif tampon (2a, 3a) est prévu pour bufferiser et fournir avec retard de temps les signaux transmis via le canal de communication (4), de telle sorte que les signaux provenant de l'interface homme-machine (2) et du téléopérateur (3) sont transmis via le canal de communication (4) de manière effective avec retard de temps et avec la même durée de temps.

**3.** Système de téléprésence selon la revendication 1 ou 2, dont l'interface homme-machine (2) est réalisée de telle sorte qu'elle soumet les premiers signaux engendrés sur la base de l'actionnement manuel de l'interface homme-machine (2) à une transformation passivante, en particulier à une transformation de dispersion pour obtenir des premiers signaux transformés et pour les transmettre au téléopérateur (3) via le canal de communication (3), le téléopérateur (3) étant prévu pour retransformer les premiers signaux transformés reçus, pour réaliser l'action de soumettre les deuxièmes signaux à une transformation passivante, en particulier à une transformation de dispersion pour obtenir des deuxièmes signaux transformés et pour les transmettre à l'interface homme-machine (2) via le canal de communication (4), et l'interface homme-machine (2) étant prévue pour retransformer les deuxièmes signaux transformés reçus.

**4.** Système de téléprésence selon l'une des revendications 1 à 3, dans lequel ledit au moins un dispositif tampon (2a, 3a) bufférise et fournit avec retard de temps les signaux transmis via le canal de communication (4).

**5.** Système de téléprésence selon l'une des revendications 1 à 4, dans lequel ledit au moins un dispositif tampon (2a, 3a) est prévu de telle manière pour bufferiser et fournir avec retard de temps les signaux transmis via le canal de communication (4) de telle sorte que les signaux provenant de l'interface homme-machine (2) et du téléopérateur sont transmis avec la même durée de temps, qui correspond à un retard de temps maximum dû au canal de communication (4), avec retard de temps via le canal de communication (4).

**6.** Système de téléprésence selon la revendication 3, dans lequel le dispositif tampon (3a) bufférise et fournit avec retard de temps les premiers signaux transformés et transmis via le canal de communication (4), de telle sorte que le téléopérateur (3) retransforme les premiers signaux transformés retardés dans le temps par le dispositif tampon (3a), et il est prévu un autre dispositif tampon (2a) qui bufférise et fournit avec retard de temps les deuxièmes signaux transformés et transmis via le canal de communication (4), de telle sorte que l'interface homme-machine (2) retransforme les deuxième signaux transformés retardés dans le temps par l'autre dispositif tampon (24).

**7.** Système de téléprésence selon la revendication 6, dans lequel le retard de temps *PT1* du dispositif tampon (3a) et le retard de temps *PT2* de l'autre dispositif tampon (2a) sont calculés de la manière suivante :

$$PT2 = Tmax - T1$$

$$PT1 = Tmax - T2,$$

*Tmax* étant au moins le retard de temps maximum du canal de communication (4) et *T1* le retard de temps du canal de communication (4) en direction interface homme-machine (2) / téléopérateur (3) et *T2* étant le retard de temps du canal de communication (4) en direction téléopérateur / interface homme-machine (2).

**8.** Système de téléprésence selon la revendication 7, qui est prévu pour déterminer les retards de temps du canal de communication (4) au moyen d'une mesure de temps sur l'interface homme-machine (2) et sur le téléopérateur (3).

**9.** Système de téléprésence selon l'une des revendications 1 à 8, qui est prévu pour déclencher la cadence du canal de communication (4) pour des signaux émis par l'interface homme-machine (2) au moyen de la cadence de l'interface homme-machine (2), pour déclencher la cadence du téléopérateur (3) au moyen de la cadence du canal de communication (4) pour des signaux émis en direction du téléopérateur (3), pour déclencher la cadence du canal de communication (4) en direction de l'interface homme-machine (2) avec la cadence du téléopérateur (3), et sur la base d'une comparaison de la cadence du canal de communication (4) pour des signaux émis en direction de l'interface homme-machine (2) et de la cadence de l'interface homme-machine (2), pour conclure un manque de synchronisation du système de téléprésence.

**10.** Système de téléprésence selon l'une des revendications 1 à 9, dans lequel le téléopérateur (3) est réalisé sous forme d'au moins un bras de robot (22, 23) avec plusieurs membres reliés par des articulations, des entraînements pour mouvoir les membres et un dispositif de fixation ainsi qu'un dispositif de commande (30) pour piloter les entraînements dudit au moins un bras de robot (22, 23).

**11.** Système de téléprésence selon l'une des revendications 1 à 10, qui est réalisé sous forme de poste de travail médical.

FIG. 1

FIG. 2

# FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010025943 A1
- US 5046022 A

- DE 102004012042 A